# EUROPEAN PATENT APPLICATION

(11) **EP 2 363 417 A1**
(43) Date of publication of application: **07.09.2011**
(21) Application number: 10180713.9
(22) Date of filing: 10.04.2007
(51) Int. Cl.: C07K 16/28

(54) **Antibodies against insulin-like growth factor I receptor and uses thereof**

(30) Priority: 11.04.2006 EP 06007571
(62) Divisional of application: 07724106.5
(71) Applicant: F. Hoffmann-La Roche AG, 4070 Basel (CH)
(72) Inventor: Hansen, Silke, 82393, Iffeldorf (DE); Kuenkele, Klaus-Peter, 83671, Benediktbeuern (DE); Reusch, Dietmar, 81475, Muenchen (DE); Schumacher, Ralf, 82377, Penzberg (DE)
(74) Representative: Burger, Alexander

(57) **Abstract**

An antibody binding to IGF-IR, being of human IgG1 or IgG3 type and being glycosylated with a sugar chain at Asn297, said antibody being characterized in that the amount of fucose within said sugar chain is at least 99%, and in addition the amount of NGNA is 1% or less and/ or the amount of N-terminal alpha-1,3-galactose is 1% or less has improved properties in antitumor therapy.

## Description

The present invention relates to antibodies against insulin-like growth factor I receptor (IGF-IR), methods for their production, pharmaceutical compositions containing said antibodies, and uses thereof.

Insulin-like growth factor I receptor (IGF-IR, EC 2.7.112, CD 221 antigen) belongs to the family of transmembrane protein tyrosine kinases (LeRoith, D., et al., Endocrin. Rev. 16 (1995) 143-163; and Adams, T.E., et al., Cell. Mol. Life Sci. 57 (2000) 1050-1093). IGF-IR binds IGF-I with high affinity and initiates the physiological response to this ligand in vivo. IGF-IR also binds to IGF-II, however with slightly lower affinity. IGF-IR overexpression promotes the neoplastic transformation of cells and there exists evidence that IGF-IR is involved in malignant transformation of cells and is therefore a useful target for the development of therapeutic agents for the treatment of cancer (Adams, T.E., et al., Cell. Mol. Life Sci. 57 (2000) 1050-1093).

Antibodies against IGF-IR are well-known in the state of the art and investigated for their antitumor effects in vitro and in vivo (Benini, S., et al., Clin. Cancer Res. 7 (2001) 1790-1797; Scotlandi, K., et al., Cancer Gene Ther. 9 (2002) 296-307; Scotlandi, K., et al., Int. J. Cancer 101 (2002) 11-16; Brunetti, A., et al., Biochem. Biophys. Res. Commun. 165 (1989) 212-218; Prigent, S.A., et al., J. Biol. Chem. 265 (1990) 9970-9977; Li, S.L., et al., Cancer Immunol. Immunother. 49 (2000) 243-252; Pessino, A., et al., Biochem. Biophys. Res. Commun. 162 (1989) 1236-1243; Surinya, K.H., et al., J. Biol. Chem. 277 (2002) 16718-16725; Soos, M.A., et al., J. Biol. Chem., 267 (1992) 12955-12963; Soos, M.A., et al., Proc. Natl. Acad. Sci. USA 86 (1989) 5217-5221; O'Brien, R.M., et al., EMBO J. 6 (1987) 4003-4010; Taylor, R., et al., Biochem. J. 242 (1987) 123-129; Soos, M.A., et al., Biochem. J. 235 (1986) 199-208; Li, S.L., et al., Biochem. Biophys. Res. Commun. 196 (1993) 92-98; Delafontaine, P., et al., J. Mol. Cell. Cardiol. 26 (1994) 1659-1673; Kull, F.C. Jr., et al. J. Biol. Chem. 258 (1983) 6561-6566; Morgan, D.O., and Roth, R.A., Biochemistry 25 (1986) 1364-1371; Forsayeth, J.R., et al., Proc. Natl. Acad. Sci. USA 84 (1987) 3448-3451; Schaefer, E.M., et al., J. Biol. Chem. 265 (1990) 13248-13253; Gustafson, T.A., and Rutter, W.J., J. Biol. Chem. 265 (1990) 18663-18667; Hoyne, P.A., et al., FEBS Lett. 469 (2000) 57-60; Tulloch, P.A., et al., J. Struct. Biol. 125 (1999) 11-18; Rohlik, Q.T., et al., Biochem. Biophys. Res. Comm. 149 (1987) 276-281; and Kalebic, T., et al., Cancer Res. 54 (1994) 5531-5534; Adams, T. E., et al., Cell. Mol. Life Sci. 57 (2000) 1050-1093; Dricu, A., et al., Glycobiology 9 (1999) 571-579; Kanter-Lewensohn, L., et al., Melanoma Res. 8 (1998) 389-397; Li, S.L., et al., Cancer Immunol. Immunother. 49 (2000) 243-252). Antibodies against IGF-IR are also described in a lot of further publications, e.g., Arteaga, C.L., et al., Breast Cancer Res. Treatment 22 (1992) 101-106; and Hailey, J., et al., Mol. Cancer Ther. 1 (2002) 1349-1353.

In particular, the monoclonal antibody against IGF-IR called αIR3 is widely used in the investigation of studying IGF-IR mediated processes and IGF-I mediated diseases such as cancer. Alpha-IR-3 was described by Kull, F.C., J. Biol. Chem. 258 (1983) 6561-6566. In the meantime, about a hundred publications have been published dealing with the investigation and therapeutic use of αIR3 in regard to its antitumor effect, alone and together with cytostatic agents such as doxorubicin and vincristine. αIR3 is a murine monoclonal antibody which is known to inhibit IGF-I binding to IGF receptor but not IGF-II binding to IGF-IR. αIR3 stimulates at high concentrations tumor cell proliferation and IGF-IR phosphorylation (Bergmann, U., et al., Cancer Res. 55 (1995) 2007-2011; Kato, H., et al., J. Biol. Chem. 268 (1993) 2655-2661). There exist other antibodies (e.g., 1H7, Li, S.L., et al., Cancer Immunol. Immunother. 49 (2000) 243-252) which inhibit IGF-II binding to IGF-IR more potently than IGF-I binding. A summary of the state of the art of antibodies and their properties and characteristics is described by Adams, T.E., et al., Cell. Mol. Life Sci. 57 (2000) 1050-1093.

Most of the antibodies described in the state of the art are of mouse origin. Such antibodies are, as is well known in the state of the art, not useful for the therapy of human patients without further alterations like chimerization or humanization. Based on these drawbacks, human antibodies are clearly preferred as therapeutic agents in the treatment of human patients. Human antibodies are well-known in the state of the art (van Dijk, M.A., and van de Winkel, J.G., Curr. Opin. Chem. Biol. 5 (2001) 368-374). Based on such technology, human antibodies against a great variety of targets can be produced. Examples of human antibodies against IGF-IR are described in WO 02/053596, WO2004071529, WO2005016967 WO2006008639, US20050249730, US20050084906, WO2005058967, WO2006013472, US20030165502, WO2005082415, WO2005016970, WO03106621, WO04083248, WO2003100008, WO2004087756, WO2005005635 and WO2005094376.

However, there is still a need for antibodies against IGF-IR with convincing benefits for patients in need of antitumor therapy. The relevant benefit for the patient is, in simple terms, reduction in tumor growth and a significant prolongation of time to progression caused by the treatment with the antitumorigenic agent.

Routier, F.H. et al., Glycoconjugate J. 14 (1997) 201-207 report the glycosylation pattern of a humanized IgG1 antibody expressed in CHO-DUKX cells. This antibody shows a molar ratio of Fuc: Man of 0.8 : 3.0, which refers to a fucosylation ratio of 80%. Niwa, R. et al., J. Immunol. Methods 306 (2005) 151-160 report for anti-CD20 IgG1 and IgG3 antibodies recombinantly produced in CHO DG44 fucosylation of 90% resp. 91%. Mimura, Y. et al., J. Immunol. Methods 247 (2001) 205-216 report that butyrate increases production of human chimeric IgG in CHO-K1 cells whilst maintaining function and glycoform profile. The oligosaccharide profiles show a considerable content of afucosylated glycan structures. Raju, T.S., BioProcess International 1 (2003) 44-53 report the impact of glycosylation variation by expression systems on the biological activity of therapeutic immunoglobulins and the nomenclature. Ma, S. et al., Anal. Chem. 71 (1999) 5185-5192 report the carbohydrate analysis of rituximab. Rituximab shows 9-10% fucosylation (Niwa, R. et al., J. Immunol. Methods 306 (2005) 151-160). Fujii, S., J. Biol. Chem 265 (1990) 6009-6018 report that bovine IgG includes about 11% afucosylated IgG. Mizuochi, T., J. Immunol. 129 (1982) 2016-2020 report that human IgG is about 14% afucosylated. Bergwerff, A.A., Glycoconjugate J. 12 (1995) 318-330 report that antibodies produced in mouse SP2/0 contains N-glycolylneuraminic acid (NGNA) oligosaccharides in large amounts. Nahrgang, S. et al., In: Animal Cell Technology: Products from Cells, Cells as Products, Bernard, A. et al. (eds.), Kluwer Academic Publishers, Dordrecht, NL, (1999) pp. 259-261, report that for CHO expression of IgG1 after transient transfection a poor overall glycosylation is found. Lund, J. et al., Mol. Immunol. 30 (1993) 741-748 report recombinant production of a mouse-human chimeric antibody in mouse transfectoma cells. The IgG1 antibody is afucosylated in an amount of 13%. Patel, T.P., et al., Biochem. J. 285 (1992) 839-845 report on glycosylation of antibodies from hybridoma cells and mouse ascites. Niwa R. et al., J. Immunol. Methods 306 (2005) 151-160, report for CD20 IgG1 antibody a fucosylation of 91% after recombinant production in CHO DG44 and Mori, K. et al., Biotech. Bioeng. 88 (2004) 901-908 a fucosylation of 94%. Davies, J., et al., Biotechnol. Bioeng. 74 (2001) 288-294 report that expression of antibodies with altered glycoforms leads to an increase of ADCC. Sheeley, D.M., et al., Anal. Biochem. 247 (1997) 102-110 compare antibody glycosylation in different expression systems. Shields, R.L., et al., J. Biol. Chem. 277 (2002) 26733-26740 report that lack of fucose on human IgG1 Fc improves FcγRIII binding and ADCC. An anti Her2 antibody being about 90% fucosylated shows also ADCC in a considerable amount. Zhu, L., et al., Nature Biotechnol. 23 (2005) 1159-1169 report on the production of human antibodies in chicken eggs.

### Summary of the Invention

The invention comprises an antibody binding to IGF-IR, being of human IgG1 or IgG3 type and being glycosylated with a sugar chain at Asn297, said antibody being characterized in that the amount of fucose within said sugar chain is at least 98% ("completely fucosylated", preferred versions see below), and in addition the amount of NGNA is 1% or less and/ or the amount of N-terminal alpha-1,3-galactose is 1% or less.

According to the invention "amount" means the amount of said sugar within the sugar chain at Asn297, related to the sum of G0, G1, G2 (without mannose(4 and 5)) as 100% and as calculated in example 3.

According to the invention it is possible to provide antibodies binding to IGF-IR with a fucosylation of even 99.4% or more, 99.5% or more or 99.9% or more.

Preferably the amount of NGNA is 0.5% or less, more preferably 0.1% or less and even not detectable by LCMS (Liquid Chromatography/Mass Spectrometry).

Preferably the amount of N-terminal alpha-1,3-galactose is 0.5% or less, more preferably 0.1% or less and even not detectable by LCMS.

The sugar chain show preferably the characteristics of N-linked glycans attached to Asn297 of an antibody binding to IGF-IR recombinantly expressed in a CHO (chinese hamster ovary) cell.

Preferably the CHO cell is a CHO cell comprising deletion (e.g. DG44) or functional inactivation of both DHFR alleles or a deletion of one DHFR allel and a functional inactivation of the second DHFR allel (e.g. DXB11).

Preferably the antibody is a monoclonal antibody. Preferably the antibody is a chimeric, humanized or human antibody.

The invention comprises preferably a completely fucosylated antibody binding to IGF-IR and inhibiting the binding of IGF-I and IGF-II to IGF-IR, characterized in that said antibody shows one or more properties selected from the group consisting of:
a) shows a ratio of IC₅₀ values of inhibition of the binding of IGF-I to IGF-IR to the inhibition of binding of IGF-II to IGF-IR of 1:3 to 3:1,
b) inhibits for at least 80%, preferably at least 90%, at a concentration of 5 nM IGF-IR phosphorylation in a cellular phosphorylation assay using HT29 cells in a medium containing 0.5% heat inactivated fetal calf serum (FCS) when compared to such an assay without said antibody,
c) shows no IGF-IR stimulating activity (no signaling , no IGF-1 mimetic activity) measured as PKB phosphorylation at a concentration of 10 µM in a cellular phosphorylation assay using 3T3 cells providing 400,000 to 600,000 molecules IGF-IR per cell in a medium containing 0.5% heat inactivated fetal calf serum (FCS) when compared to such an assay without said antibody,
d) downregulates 50% or more of IGF-1R expressed on a tumor cell (e.g. HT29) 24h after addition of the antibody to the cell.

Antibodies according to the invention show benefits for patients in need of antitumor therapy and provide reduction of tumor growth and a significant prolongation of the time to progression. The antibodies according to the invention have new and inventive properties causing a benefit for a patient suffering from a disease associated with an IGF deregulation, especially a tumor disease. The antibodies according to the invention are characterized by the abovementioned properties.

Surprisingly an antibody according to the invention ("completely fucosylated antibody") does not cause ADCC (antibody-dependent cell-mediated cytotoxicity) (within 3×SD (standard deviation) from reference standard antibody (antibody against keyhole limpet hemocyanin, KLH antibody)) as shown in the ADCC assay described in example).

Preferably the antibody is specific binding to IGF-IR, inhibiting the binding of IGF-I and IGF-II to IGF-IR at the abovementioned ratio, is of IgG1 isotype, and is not activating the IGF-IR signaling even in IGF-IR overexpressing cells at a 200-fold concentration of its IC₅₀ value.

Antibodies binding to IGF-1R, having no "IGF-I mimetic activity" in combination with "complete fucosylation" provide a strong advantage when used as a therapeutic agent.

Preferably, at a concentration of 5 nM the antibodies according to the invention completely inhibit IGF-I mediated signal transduction of IGF-IR in tumor cells.

Preferred nucleic acids of polypeptides which are capable of assembling together with the respective other antibody chain to an antibody according to the invention are defined below:
a) an antibody heavy chain comprising as CDRs CDR1 (aa 31-35), CDR2 (aa 50-66) and CDR3 (aa 99-107) of SEQ ID NO:1 or 3;
b) an antibody light chain comprising as CDRs CDR1 (aa 24-34), CDR2 (aa 50-56) and CDR3 (aa 89-98) of SEQ ID NO:2 or 4.

The antibody is preferably a monoclonal antibody and, in addition, a chimeric antibody (human constant chain), a humanized antibody and especially preferably a human antibody.

The antibody preferably binds to IGF-IR human (EC 2.7.1.112, SwissProt P08069) in competition to antibody 18.

The antibody is preferably further characterized by an affinity of 10⁻⁸ M (K_{D}) or less, preferably of about 10⁻⁹ to 10⁻¹³ M.

The antibody shows preferably no detectable concentration dependent inhibition of insulin binding to the insulin receptor.

The antibody is preferably of IgG 1 type.

The antibody according to the invention considerably prolongates the time to progression in relevant xenograft tumor models in comparison with vehicle treated animals and reduces tumor growth. The antibody inhibits the binding of IGF-I and IGF-II to IGF-IR in vitro and in vivo, preferably in about an equal manner for IGF-I and IGF-II.

Preferably, the antibodies according to the invention comprise as complementarity determining regions (CDRs) the following sequences:
a) an antibody heavy chain comprising as CDRs CDR1 (aa 31-35), CDR2 (aa 50-66) and CDR3 (aa 99-107) of SEQ ID NO:1 or 3;
b) an antibody light chain comprising as CDRs CDR1 (aa 24-34), CDR2 (aa 50-56) and CDR3 (aa 89-98) of SEQ ID NO:2 or 4.

Preferred variable regions and CDRs, especially CDR3 of heavy chain of antibodies according to the invention are provided by <IGF-1R> HUMAB Clone 18 (antibody 18) and <IGF-1R> HUMAB Clone 22 (antibody 22), deposited with Deutsche Sammlung von Mikroorganismen und Zellkulturen GmbH (DSMZ), Germany.

| **Cell line** | **Deposition No.** | **Date of deposit** |
|---|---|---|
| <IGF-1R> HUMAB-Clone 18 | DSM ACC 2587 | 10.04.2003 |
| <IGF-1R> HUMAB-Clone 22 | DSM ACC 2594 | 09.05.2003 |

These antibodies are described in detail in WO 2005/005635.

Further preferred variable regions and CDRs, especially CDR3 of heavy chain of antibodies according to the invention are provided by <IGF-1R> HuMab Clone 1a (antibody 1A, Ab 1A or Ak 1A), <IGF-1R> HuMab Clone 23 (antibody 23), and <IGF-1R> HuMab-Clone 8 (antibody 8), deposited with Deutsche Sammlung von Mikroorganismen und Zellkulturen GmbH (DSMZ), Germany:

| **Cell line** | **Deposition No.** | **Date of Deposit** |
|---|---|---|
| <IGF-1R> HUMAB Clone 1a | DSM ACC 2586 | 10.04.2003 |
| <IGF-1R> HUMAB Clone 23 | DSM ACC 2588 | 10.04.2003 |
| <IGF-1R> HUMAB-Clone 8 | DSM ACC 2589 | 24.04.2003 |

These antibodies are described in detail in WO 2004/087756.

The invention further provides methods for the recombinant production of such antibodies.

The invention further provides methods for treating cancer, comprising administering to a patient diagnosed as having cancer (and therefore being in need of an antitumor therapy) an effective amount of an antagonistic antibody against IGF-IR according to the invention. The antibody may be administered alone, in a pharmaceutical composition, or alternatively in combination with a cytotoxic treatment such as radiotherapy or a cytotoxic agent or a prodrug thereof.

The invention further comprises the use of an antibody according to the invention for cancer treatment and for the manufacture of a pharmaceutical composition according to the invention. In addition, the invention comprises a method for the manufacture of a pharmaceutical composition according to the invention.

The invention further comprises a pharmaceutical composition containing an antibody according to the invention in a pharmaceutically effective amount, optionally together with a buffer and/or an adjuvant useful for the formulation of antibodies for pharmaceutical purposes.

The invention further provides pharmaceutical compositions comprising such antibodies in a pharmaceutically acceptable carrier. In one embodiment, the pharmaceutical composition may be included in an article of manufacture or kit.

The invention further comprises a method for the production of a recombinant human antibody according to the invention, characterized by expressing a nucleic acid encoding an antibody binding to IGF-1R in a CHO host cell, which completely fucosylates said antibody and recovering said antibody from said cell. The invention further comprises the antibody obtainable by such a recombinant method.

### Brief Description of the Drawing

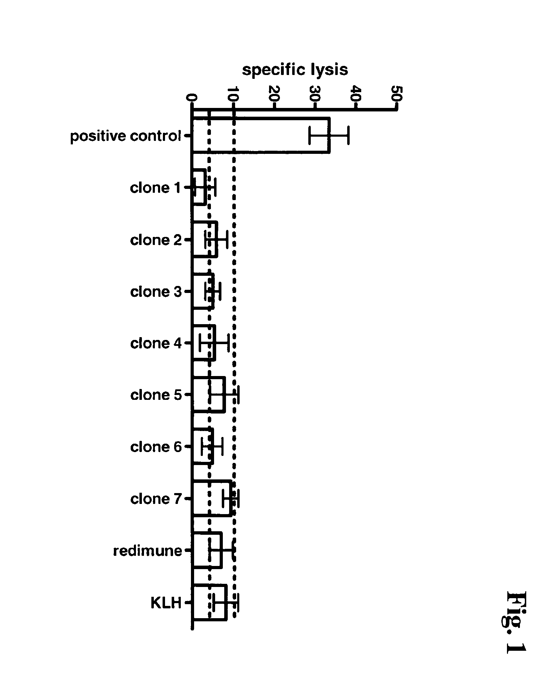

Figure 1 is a bar chart showing the ADCC activity or lack thereof in antibodies of the invention and in control and comparative antibodies.

### Detailed Description of the Invention

The term "antibody" encompasses the various forms of antibodies including but not being limited to whole antibodies, antibody fragments, human antibodies, humanized antibodies and genetically engineered antibodies as long as the characteristic properties according to the invention are retained.

"Antibody fragments" comprise a portion of a full length antibody, generally at least the antigen binding portion or the variable region thereof. Examples of antibody fragments include diabodies, single-chain antibody molecules, immunotoxins, and multispecific antibodies formed from antibody fragments.

The terms "monoclonal antibody" or "monoclonal antibody composition" as used herein refer to a preparation of antibody molecules of a single amino acid composition. Accordingly, the term "human monoclonal antibody" refers to antibodies displaying a single binding specificity which have variable and constant regions derived from human germline immunoglobulin sequences.

The term "chimeric antibody" refers to a monoclonal antibody comprising a variable region, i.e., binding region, from one source or species and at least a portion of a constant region derived from a different source or species, usually prepared by recombinant DNA techniques. Chimeric antibodies comprising a murine variable region and a human constant region are especially preferred. Such murine/human chimeric antibodies are the product of expressed immunoglobulin genes comprising DNA segments encoding murine immunoglobulin variable regions and DNA segments encoding human immunoglobulin constant regions. Other forms of "chimeric antibodies" encompassed by the present invention are those in which the class or subclass has been modified or changed from that of the original antibody. Such "chimeric" antibodies are also referred to as "class-switched antibodies." Methods for producing chimeric antibodies involve conventional recombinant DNA and gene transfection techniques now well known in the art. See, e.g., Morrison, S.L., et al., Proc. Natl. Acad Sci. USA 81 (1984) 6851-6855; US Patent Nos. 5,202,238 and 5,204,244.

The term "humanized antibody" refers to antibodies in which the framework or "complementarity determining regions" (CDR) have been modified to comprise the CDR of an immunoglobulin of different specificity as compared to that of the parent immunoglobulin. In a preferred embodiment, a murine CDR is grafted into the framework region of a human antibody to prepare the "humanized antibody." See, e.g., Riechmann, L., et al., Nature 332 (1988) 323-327; and Neuberger, M.S., et al., Nature 314 (1985) 268-270. Particularly preferred CDRs correspond to those representing sequences recognizing the antigens noted above for chimeric and bifunctional antibodies.

The term "human antibody", as used herein, is intended to include antibodies having variable and constant regions derived from human germline immunoglobulin sequences. The variable heavy chain is preferably derived from germline sequence DP-50 (GenBank LO6618) and the variable light chain is preferably derived from germline sequence L6 (GenBank X01668) or the variable heavy chain is preferably derived DP-61 (GenBank M99682) and the variable light chain is derived from germline sequence L15 (GenBank K01323). The constant regions of the antibody are constant regions of human IgG1 type. Such regions can be allotypic and are described by, e.g., Johnson, G., and Wu, T.T., Nucleic Acids Res. 28 (2000) 214-218 and the databases referenced therein.

The term "recombinant human antibody", refers to antibodies having variable and constant regions derived from human germline immunoglobulin sequences in a rearranged form. The recombinant human antibodies according to the invention have been subjected to in vivo somatic hypermutation. Thus, the amino acid sequences of the VH and VL regions of the recombinant antibodies are sequences that, while derived from and related to human germline VH and VL sequences, may not naturally exist within the human antibody germline repertoire in vivo.

As used herein, " binding" refers to antibody binding to IGF-IR with an affinity of about 10⁻¹³ to 10⁻⁸M (K_{D}), preferably of about 10⁻¹³ to 10⁻⁹ M.

The term "nucleic acid molecule", as used herein, is intended to include DNA molecules and RNA molecules. A nucleic acid molecule may be single-stranded or double-stranded, but preferably is double-stranded DNA.

Human constant domains having of IgG1 or IgG3 type are described in detail by Kabat, E.A. et al., Sequences of Proteins of Immunological Interest, 5th ed., Public Health Service, National Institutes of Health, Bethesda, MD. (1991), and by Brüggemann, M., et al., J. Exp. Med. 166 (1987) 1351-1361; Love, T.W., et al., Methods Enzymol. 178 (1989) 515-527. Examples are shown in SEQ ID NOS:5 to 8. Other useful and preferred constant domains are the constant domains of the antibodies obtainable from the hybridoma cell lines deposited with DSMZ for this invention.

Constant domains of IgG1 or IgG3 type are glycosylated at Asn297. "Asn 297" according to the invention means amino acid asparagine located at about position 297 in the Fc region; based on minor sequence variations of antibodies, Asn297 can also be located some amino acids (usually not more than ±3 amino acids) upstream or downstream. For example, in one antibody according to the invention "Asn297" is located at amino acid position 298.

Glycosylation of human IgG1 or IgG3 occurs at Asn297 as core fucosylated bianntennary complex oligosaccharide glycosylation terminated with up to 2 Gal (galactose) residues. These structures are designated as G0, G1 (α1,6 or α1,3) or G2 glycan residues, depending from the amount of terminal Gal residues (Raju, T.S., BioProcess Int. 1 (2003) 44-53). CHO type glycosylation of antibody Fc parts is e.g. described by Routier, F.H., Glycoconjugate J. 14 (1997) 201-207.

The "variable region" (variable region of a light chain (VL), variable region of a heavy chain (VH)) as used herein denotes each of the pair of light and heavy chains which is involved directly in binding the antibody to the antigen. The domains of variable human light and heavy chains have the same general structure and each domain comprises four framework (FR) regions whose sequences are widely conserved, connected by three "hypervariable regions" (or complementarity determining regions, CDRs). The framework regions adopt a β-sheet conformation and the CDRs may form loops connecting the β-sheet structure. The CDRs in each chain are held in their three-dimensional structure by the framework regions and form together with the CDRs from the other chain the antigen binding site. The antibody heavy and light chain CDR3 regions play a particularly important role in the binding specificity/affinity of the antibodies according to the invention and therefore provide a further object of the invention.

The terms "hypervariable region" or "antigen-binding portion of an antibody" when used herein refer to the amino acid residues of an antibody which are responsible for antigen-binding. The hypervariable region comprises amino acid residues from the "complementarity determining regions" or "CDRs". "Framework" or "FR" regions are those variable domain regions other than the hypervariable region residues as herein defined. Therefore, the light and heavy chains of an antibody comprise from N- to C-terminus the domains FR1, CDR1, FR2, CDR2, FR3, CDR3, and FR4. Especially, CDR3 of the heavy chain is the region which contributes most to antigen binding. CDR and FR regions are determined according to the standard definition of Kabat, E.A. et al., Sequences of Proteins of Immunological Interest, 5th ed., Public Health Service, National Institutes of Health, Bethesda, MD. (1991)) and/or those residues from a "hypervariable loop".

The term "binding to IGF-IR" as used herein means the binding of the antibody to IGF-IR in an in vitro assay, preferably in a binding assay in which the antibody is bound to a surface and binding of IGF-IR is measured by Surface Plasmon Resonance (SPR). Binding means a binding affinity (K_{D}) of 10⁻⁸ M or less, preferably 10⁻¹³ to 10⁻⁹ M.

Binding to IGF-IR can be investigated by a BIAcore assay (Pharmacia Biosensor AB, Uppsala, Sweden). The affinity of the binding is defined by the terms ka (rate constant for the association of the antibody from the antibody/antigen complex), kd (dissociation constant), and K_{D} (kd/ka). The antibodies according to the invention show a K_{D} of 10⁻¹⁰ M or less.

The binding of IGF-I and IGF-II to IGF-IR is also inhibited by the antibodies according to the invention. The inhibition is measured as IC₅₀ in an assay for binding of IGF-I/IGF-II to IGF-IR on tumor cells. Such an assay is described in Example 7. In such an assay, the amount of radiolabeled IGF-I or IGF-II or IGF-IR binding fragments thereof bound to the IGF-IR provided at the surface of said tumor cells (e.g. HT29) is measured without and with increasing concentrations of the antibody. The IC₅₀ values of the antibodies according to the invention for the binding of IGF-I and IGF-II to IGF-IR are no more than 2 nM and the ratio of the IC₅₀ values for binding of IGF-I/IGF-II to IGF-IR is about 1:3 to 3:1. IC₅₀ values are measured as average or median values of at least three independent measurements. Single IC₅₀ values may be out of the scope.

The term "inhibiting the binding of IGF-I and IGF-II to IGF-IR" as used herein refers to inhibiting the binding of I¹²⁵-labeled IGF-I or IGF-II to IGF-IR presented on the surface of HT29 (ATCC HTB-38) tumor cells in an in vitro assay. Inhibiting means an IC₅₀ value of 2 nM or lower.

The term "IGF-IR expressing cells" refers to such cells which are overexpressing IGF-I receptor to about at least 20,000 receptors/cell. Such cells are, for example, tumor cell lines such as NCI H322M or HT29, or a cell line (e.g. 3T3 ATCC CRL1658) overexpressing IGF-IR after transfection with an expression vector for IGF-IR. The amount of receptors per cell is measured according to Lammers, R., et al., EMBO J. 8 (1989) 1369-1375.

The term "inhibiting of IGF-IR phosphorylation" refers to a cellular phosphorylation assay using 3T3 cells providing 400,000 to 600,000 molecules IGF-IR per cell in a medium containing 0.5% heat inactivated fetal calf serum (FCS) when compared to such an assay without said antibody. Phosphorylation is detected by Western blotting using an antibody specific for tyrosine-phosphorylated proteins. Such an assay is described in Example 11. Heat inactivation of FCS is performed by short term heating to 56° C for inactivation of the complement system.

The term "inhibiting of PKB phosphorylation" refers to a cellular phosphorylation assay using 3T3 cells providing 400,000 to 600,000 molecules IGF-IR per cell in a medium containing 0.5% heat inactivated fetal calf serum (FCS) when compared to such an assay without said antibody. Phosphorylation is detected by Western blotting using an antibody specific for PKB phosphoylated at serine 473 of PKB (Akt 1, Swiss Prot Acc. No. P31749). Such an assay is described in Example 11.

The term "antibody-dependent cellular cytotoxicity (ADCC)" refers to lysis of human tumor target cells by an antibody according to the invention in the presence of effector cells. ADCC is measured preferably by the treatment of a preparation of IGF-IR expressing cells with an antibody according to the invention in the presence of effector cells such as freshly isolated PBMC (peripheral blood mononuclear cells) or purified effector cells from buffy coats, like monocytes or NK cells (natural killer cells).

The term "complete inhibition of IGF-I mediated signal transduction" refers to the inhibition of IGF-I-mediated phosphorylation of IGF-IR. For such an assay, IGF-IR expressing cells, preferably H322M cells, are stimulated with IGF-I and treated with an antibody according to the invention (an antibody concentration of 5 nM or higher is useful). Subsequently, an SDS PAGE is performed and phosphorylation of IGF-IR is measured by Western blotting analysis with an antibody specific for phosphorylated tyrosine. Complete inhibition of the signal transduction is found if on the Western blot visibly no band can be detected which refers to phosphorylated IGF-IR.

The antibodies according to the invention show preferably a binding to the same epitope of IGF-IR as antibody 18 or are inhibited in binding to IGF-IR due to steric hindrance of binding by antibody 18. Binding inhibition can be detected by an SPR assay using immobilized antibody 18 and IGF-IR at a concentration of 20-50 nM and the antibody to be detected at a concentration of 100 nM. A signal reduction of 50% or more shows that the antibody competes with antibody 18. Such an assay can be performed in the same manner by using antibody 22 as an immobilized antibody.

The term "epitope" means a protein determinant capable of specific binding to an antibody. Epitopes usually consist of chemically active surface groupings of molecules such as amino acids or sugar side chains and usually have specific three dimensional structural characteristics, as well as specific charge characteristics.

Conformational and nonconformational epitopes are distinguished in that the binding to the former but not the latter is lost in the presence of denaturing solvents.

The antibodies according to the invention inhibit IGF-IR phosphorylation of tyrosine and preferably also PKB phosphorylation of tyrosine to a similar extent.

The antibodies according to the invention preferably downregulate the IGF-IR protein level in tumor cells and in tumors , e.g. xenograft tumors.

The antibodies according to the invention inhibit preferably the three-dimensional growth of tumor cells in a colony formation assay as well as proliferation of IGF-IR expressing cells (e.g. NIH 3T3 cells).

The antibodies according to the invention preferably do not inhibit binding of insulin to insulin receptor in a binding competition assay on insulin receptor overexpressing 3T3 cells using the antibody in a concentration of 200 nmol/l.

The antibodies according to the invention are produced by recombinant means in a CHO cell which completely fucosylate the antibody. For the protein expression, nucleic acids encoding light and heavy chains or fragments thereof are inserted into expression vectors by standard methods. Expression is performed in such CHO cells, and the antibody is recovered from the cells (supernatant or cells after lysis).

A useful CHO host cell can be produced by a method comprising cultivating a CHO cell, transfected with nucleic acid encoding an antibody according to the invention, under DHFR selection pressure, picking single clones expanding the clones and selecting a clone producing an antibody with the glycosylation pattern according to the invention. Preferably cultivation is performed for at least two, preferably at least three weeks. The CHO cell is preferably a DG44 cell.

The term "CHO cell" encompasses the various forms of Chinese Hamster Ovary (CHO) cells based on two functionally inactive, preferably deleted, dhfr alleles (dihydrofolate reductase deficient (dhfr⁻)). Such dhfr⁻ cells and methods for their generation are described e.g. in Urlaub, G. et al., Cell 33 (1983) 405-412; Chasin, L. et al., Som. Cell Molec. Genet. 12 (1986) 555-556; Kolkekar, A.S. et al., Biochemistry 36 (1997) 10901-10909. Preferably the cell is a DG44 cell line. Such CHO dhfr⁻ cells can be produced using gamma rays to eliminate the entire dhfr locus. In non-mutated, wild-type cells, dhfr is an essential enzyme for de novo synthesis of glycine, purines, and thymidylate. This allows the dhfr gene encoded on plasmids to be used as a dominant selectable marker and a gene amplifier for the expression of proteins in dhfr- deficient cell lines. The dhfr⁻ mutation in DG44 cells is stable and irreversible. CHO cells successfully co-transfected with expression vector(s) for an antibody of human IgG1 or IgG3 type and the DHFR gene will possess the dhfr+ phenotype and can readily be selected by culturing the colonies on media devoid of thymidine and hypoxanthine and optionally containing methotrexate (MTX) for amplification.

DG44 cells are well known in the state of the art and e. g. commercial available as cell lines e.g. from Invitrogen Corp.(USA). DG44 cells can grow adherent, in suspension and/or in serum-free medium. As used herein, the expressions "cell," "cell line," and "cell culture" are used interchangeably and all such designations of CHO dhfr⁻ cell lines (two deleted dhfr alleles) include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Variant progeny that have the glycosylation properties according to the invention as screened for in the originally transformed cell are included.

Preferably the CHO dhfr- cell line is co-amplified with at least DHFR as one selectable marker gene. For example a mammalian expression vector containing the selectable marker(s) and the antibody gene are co-transfected into recipient CHO cells. The resulting colonies may be selected and colonies exhibiting the expected phenotype are capable of expressing the antibody. Additional selectable markers are or may not be of a dominant nature. Examples of additional selectable markers for use co-transfection include adenosine deaminase (Kaufman, R.J., et al., Proc. Natl. Acad. Sci. USA 83 (1986) 3136-3140) asparagine synthetase (Cartier, M., et al., Mol.Cell Biol. 7 (1987) 1623-1628), E. coli trpB gene and Salmonella hisD gene (Hartman, S.C., and Mulligan, R.C., Proc. Natl. Acad. Sci. USA 85 (1988) 8047-8051), M2 mouse ribonucleotide reductase (Thelander, M., and Thelander, L., EMBO J. 8 (1989) 2475-2479), human multidrug resistance gene (Kane, S.E., et al., Gene 84 (1989) 439-446), glutamine synthetase (Bebbington, C.R. et al., DNA Cloning, Vol. III, D.M. Glover (ed.), IRL Press, pp. 163-188, 1987), xanthine guanine phosphoribosyl transferase (gpt) (Mulligan, R.C., and Berg, P., Science 209 (1980) 1422-1427), hygromycin B (Santerre, R.F., et al., Gene 30 (1984) 147-156), neomycin gene (Southern, P.J., and Berg, P., J. Mol. Appl.Genet. 1 (1982) 327-341).

The selectable markers may also provide the basis upon which the genes encoding the antibody may be amplified. In co-transfection of a CHO cell line, the vector DNAs are often integrated into the chromosome of the cell at the same locus. Thus, the use of only one of the selectable markers as the basis for amplification normally results in a parallel increase in the copy number of both genes. One particular selectable marker for use in this way is dhfr which enables the desired amplification to be obtained through the use of increasing concentrations of MTX (methotrexate). A second preferred selectable marker is GS which allows amplification by the addition of methionine sulphoximine (MSX).

The selectable markers are of course under the control of regulatory elements of DNA so as to provide for their expression. In the case of the use of dhfr as a selectable marker, the regulatory elements are preferably of a viral source, such as from DNA tumor viruses. Particularly preferred are the use of an SV40 or adenovirus major late promoter. It is particularly advantageous in this regard to remove the enhancer element from the promoter thus effectively "crippling" it. This modification allows for increased levels of gene amplification at each concentration of methotrexate selection than would otherwise occur if a strong promoter was used. In the case of the use of neomycin as a selectable marker, an example of a suitable promoter is the mouse metallothionein promoter.

The general methods for recombinant production of antibodies are well-known in the state of the art and described, for example, in the review articles of Makrides, S.C., Protein Expr. Purif. 17 (1999) 183-202; Geisse, S., et al., Protein Expr. Purif. 8 (1996) 271-282; Kaufman, R.J., Mol. Biotechnol. 16 (2000) 151-161; Werner, R.G., Drug Res. 48 (1998) 870-880.

The antibodies may be present in whole cells, in the supernant, in a cell lysate, or in a partially purified or substantially pure form. Purification is performed in order to eliminate other cellular components or other contaminants, e.g. other cellular nucleic acids or proteins, by standard techniques, including alkaline/SDS treatment, CsCl banding, column chromatography, agarose gel electrophoresis, and others well known in the art. See Ausubel, F., et al., ed. Current Protocols in Molecular Biology, Greene Publishing and Wiley Interscience, New York (1987).

The control sequences that are suitable for prokaryotes, for example, include a promoter, optionally an operator sequence, and a ribosome binding site.

Eukaryotic cells are known to utilize promoters, enhancers and polyadenylation signals.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading frame. However, enhancers do not have to be contiguous. Linking is accomplished by ligation at convenient restriction sites. If such sites do not exist, the synthetic oligonucleotide adaptors or linkers are used in accordance with conventional practice.

The monoclonal antibodies can be suitably separated from a hybridoma culture medium by conventional immunoglobulin purification procedures such as, for example, protein A-Sepharose, hydroxylapatite chromatography, gel electrophoresis, dialysis, or affinity chromatography. DNA and RNA encoding the monoclonal antibodies is readily isolated from the hybridoma and sequenced using conventional procedures. The hybridoma cells can serve as a source of such DNA and RNA. Once identified and isolated, the DNA may be inserted into expression vectors, which are then transfected into CHO cells that do not otherwise produce immunoglobulin protein, to obtain the synthesis of recombinant monoclonal antibodies in the host cells.

The invention also pertains to immunoconjugates comprising the antibody according to the invention conjugated to a cytotoxic agent such as a chemotherapeutic agent, toxin (e.g., an enzymatically active toxin of bacterial, fungal, plant or animal origin, or fragments thereof), a radioactive isotope (i.e., a radioconjugate) or a prodrug of a cytotoxic agent. Agents useful in the generation of such immunoconjugates have been described above. Enzymatically active toxins and fragments thereof which can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleuritesfordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes.

Conjugates of the antibody and cytotoxic agent are made using a variety of bifunctional protein coupling agents such as N-succinimidyl-3-(2-pyridyldithiol) propionate (SPDP), iminothiolane (IT), bifunctional derivatives of imidoesters; (such as dimethyl adipimidate HCL), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediatnine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta, E.S., et al., Science 238 (1987) 1098-1104). Carbon-14-labeled 1-isothiocyanatobenzyl-3-methyldiethylene triaminepentaacetic acid (MX-DTPA) is an exemplary chelating agent for conjugation of radionucleotide to the antibody. See WO 94/11026.

In another aspect, the present invention provides a composition, e.g. a pharmaceutical composition, containing an antibody of the present invention, formulated together with a pharmaceutically acceptable carrier.

Pharmaceutical compositions of the invention also can be administered in combination therapy, i.e., combined with other agents. For example, the combination therapy can include a composition of the present invention with at least one anti-tumor agent, like a chemotherapeutic agent, a cytotoxic agent or a prodrug or other conventional therapy.

A "chemotherapeutic agent" is a chemical compound useful in the treatment of cancer. Examples of chemotherapeutic agents include Adriamycin, Doxorubicin, 5-Fluorouracil, Cytosine arabinoside ("Ara-C"), Cyclophosphamide, Thiotepa, Taxotere (docetaxel), Busulfan, Gemcitabine, Cytoxin, Taxol, Methotrexate, Cisplatin, Melphalan, Vinblastine, Bleomycin, Etoposide, Ifosfamide, Mitomycin C, Mitoxantrone, Vincreistine, Vinorelbine, Carboplatin, Teniposide, Daunomycin, Carminomycin, Aminopterin, Dactinomycin, Mitomycins, Esperamicins (see US Patent No. 4,675,187), Melphalan and other related nitrogen mustards.

The term "cytotoxic agent" as used herein refers to a substance that inhibits or prevents the function of cells and/or causes destruction of cells. The term is intended to include radioactive isotopes, chemotherapeutic agents, and toxins such as enzymatically active toxins of bacterial fungal, plant or animal origin, or fragments thereof.

The term "prodrug" as used in this application refers to a precursor or derivative form of a pharmaceutically active substance that is less cytotoxic to tumor cells compared to the parent drug and is capable of being enzymatically activated or converted into the more active parent form. See, e.g., Wilman, D.E., Biochemical Society Transactions 14 (1986) 375-382, and Stella, V.J. et al., "Prodrugs: A Chemical Approach to Targeted Drug Delivery," In: Directed Drug Delivery, Borchardt, R.T. et al., (eds.), pp. 247-267, Humana Press, Clifton, New Jersey (1985). The prodrugs of this invention include, but are not limited to, phosphate-containing prodrugs, thiophosphate- containing prodrugs, sulfate-containing prodrugs, peptide-containing prodrugs, D-amino acid-modified prodrugs, glycosylated prodrugs, β-lactam ring prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this invention include, but are not limited to, those chemotherapeutic agents described above.

As used herein, "pharmaceutically acceptable carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g. by injection or infusion).

A "pharmaceutically acceptable salt" refers to a salt that retains the desired biological activity of the antibody and does not impart any undesired toxicological effects (see e.g. Berge, S.M., et al., J. Pharm. Sci. 66 (1977) 1-19). Such salts are included in the invention. Examples of such salts include acid addition salts and base addition salts. Acid addition salts include those derived from nontoxic inorganic acids, such as hydrochloric salts.

A composition of the present invention can be administered by a variety of methods known in the art. As will be appreciated by the skilled artisan, the route and/or mode of administration will vary depending upon the desired results.

To administer a compound of the invention by certain routes of administration, it may be necessary to coat the compound with or co- administer the compound with, a material to prevent its inactivation. For example, the compound may be administered to a subject in an appropriate carrier, for example, liposomes, or a diluent. Pharmaceutically acceptable diluents include saline and aqueous buffer solutions.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

These compositions may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

The composition must be sterile and fluid to the extent that the composition is deliverable by syringe. In addition to water, the carrier preferably is an isotonic buffered saline solution.

Proper fluidity can be maintained, for example, by use of coating such as lecithin, by maintenance of required particle size in the case of dispersion and by use of surfactants. In many cases, it is preferable to include isotonic agents, for example, sugars, polyalcohols such as mannitol or sorbitol, and sodium chloride in the composition.

Preferably a completely fucosylated antibody according to the invention is useful for the treatment of NSCLC (non-small cell lung carcinoma), preferably in combination with Erlotinib (Tarceva®), for the treatment of breast cancer, preferably in combination with Herceptin® (Trastuzumab), and pancreatic tumors, preferably in combination with gemcitabine (Gemzar®).

The following examples, figure and sequence listing are provided to aid the understanding of the present invention, the true scope of which is set forth in the appended claims. It is understood that modifications can be made in the procedures set forth without departing from the spirit of the invention.

### Examples

### Cell lines

The parental cell line used for the generation of a cell line for recombinant IgG expression is a Chinese hamster ovarian (CHO) cell line, CHO-DG44 (Flintoff, W.F. et al., Somat. Cell Genet. 2 (1976) 245-261; Flintoff et al., Mol. Cell. Biol. 2 (1982) 275-285; Urlaub, G. et al., Cell 33 (1983) 405-412; Urlaub, G. et al., Somat. Cell Mol. Genet. 12 (1986) 555-566). CHO-DG44 cells have lost both endogenous loci for the enzyme Dihydrofolate Reductase (DHFR).

CHO-DG44 cells were grown in MEM alpha Minus Medium (Gibco No. 22561), 10% dialysed FCS (Gibco No. 26400-044) and 2 mmol/L L-Glutamine, 100µM Hypoxanthin, 16µM Thymidin (HT supplement).

### Plasmids

The expression system comprised the CMV promoter and is described in table 1. As antibody an antibody against IGF-1R (WO2005005635; AK18 or AK22) was used.

**Table 1**

| Bp | Vector element / DNA segment |
|---|---|
| 1-26 | Unique restriction sites: SgrAI, Sse83871 |
| 27-614 | **Human cytomegalovirus (HCMV) promoter (CMV-Prom)** including human CMV IE promoter including synthetic 5'-UTR |
| 615-641 | Linker |
| 642-780 | **Murine Ig heavy chain leader sequence (L1, signal sequence intron, L2)** |
| 642-686 | L1 |
| 687-768 | Signal intron (SS intron) |
| 769-780 | L2 |
| 781-1105 | **Variable κ-light chain domain of IGF-1R antibody (AK18)** |
| 1106-1140 | Linker |
| **1141-3134** | **Human/mouse κ-light chain hybrid intron 2** |
| 2433-2913 | κ-enhancer fragment |
| 3135-3475 | Linker |
| **3476-3795** | **κ-Light chain constant region (C-kappa)** |
| **3796-4098** | **Human Ig κ-light chain polyadenylation sequence (C-kappa pA)** |
| 4099-4137 | Linker |
| **4138-5800** | **Hygromycin resistance** |
| 4138-4485 | SV40 promoter (SV40 Prom) incl. 72bp repeat, TATA, SV40 origin |
| 4486-4502 | Linker |
| 5403-5528 | Hygromycin-B-phosphotransferase (Hyg) |
| 5529-5535 | Linker |
| 5536-5795 | SV40 polyadenylation signal (SV40 pA) |
| 5796-5800 | Linker |
| **5801-6944** | **Murine dihydrofolate reductase (DHFR)** |
| 5801-6088 | SV40 promoter (SV40 Prom) incl. 72bp repeat shortened, SV40 origin |
| 6089-6105 | Linker |
| 6106-6672 | Murine DHFR gene (murine DHFR) |
| 6673-6679 | Linker |
| 6680-6944 | SV40 polyadenylation signal (SV40 pA) |
| 6945-7181 | Linker |
| **7182-8941** | **Bacterial origin of replication and selective marker derived from plasmid pUC18** |
| 7182-7792 | Origin of replication ("pUC origin") |
| 7793-7939 | Linker |
| 7940-8847 | β-Lactamase gene (Ap(r)) |
| 8848-8941 | Linker |
| **8942-9529** | **Human cytomegalovirus (HCMV) promoter (CMV-Prom)** including human CMV IE promoter including synthetic 5'-UTR |
| 9530-9556 | Linker |
| 9557-9696 | **Murine Ig heavy chain leader sequence (L1, signal sequence intron, L2)** |
| 9557-9602 | L1 |
| 9603-9685 | Signal intron (SS intron) |
| 9686-9696 | L2 |
| 9697-10051 | **Variable IgG1 heavy chain domain of IGF-1R antibody (AK18)** |
| 10052-10085 | Linker |
| **10086-11682** | **Human/mouse heavy chain hybrid intron 2** including the part of the mouse Ig heavy chain J-segment region including the Ig heavy chain enhancer element (part JH₃, JH₄) Mouse Ig heavy chain enhancer element |
| 11683-11909 | Linker |
| **11910-13504** | **Human IgG1 heavy chain constant region (CH₁-Hinge-CH₂-CH₃)** |
| 11910-12203 | CH1 |
| 12594-12638 | Hinge |
| 12757-13086 | CH2 |
| 13184-13504 | CH3 (alternative splice site deleted) |
| 13505-13967 | **Human IgG1 heavy chain polyadenylation sequence (IgG1 pA)** |
| 13968-13970 | SgrAI-Linker |

### Example 1

### Transfection and Selection

Transfection of the expression plasmid was carried out with Fugene (Roche Diagnostics GmbH). A day after transfection, DG44 cells were put under selection pressure consisting of MEM alpha Minus Medium, 10% dialysed FCS and 2 mmol/L L-Glutamine and 20nM Methotrexate (MTX). After 3 weeks under selection pressure, single clones were picked from the plate and expanded.

Supernatants were collected and the presence of the antibody was analyzed with a human IgG-specific ELISA. Subclones were further expanded and analyzed for specific antibody production.

Clones were adapted to growth in suspension culture and serum-free medium, HyQ SFM4 CHO-Utility (HyClone #SH30516) containing 20nM MTX. In parallel, the glycopattern profile was determined. Subclones were selected providing defucosylation of 2.0% or lower ( referring to total molar oligosaccharide amount).

### Example 2

### Cultivation and Purification

3x10⁵ cells were grown in 125ml shake flasks (Corning) filled with 30ml medium at 37°C, 5% CO2, 100rpm for 10 days. Cell density was measured by CASY Counter and supernatant was taken for determination of antibody concentration by protein A affinity chromatography. About 20ml of each supernatant was purified for further biochemical characterization by Protein A chromatography (equilibration with PBS, wash with 25mM sodium citrate buffer pH 5.2, elution with 100mM sodium citrate buffer pH 2.8, CIP with 10mM NaOH).

### Example 3

### Analysis of glycostructure of antibody

Purified antibody material was analyzed by Liquid Chromatography/Mass Spectrometry (LCMS) Peptide map analysis. Samples were reduced (0.4M TRIS/HCl, 8M Guanidine/HCl, pH 8.5, DTT (3mg/ml), carboxymethylated (iodoacetic acid) and cleaved with trypsin. The peptide - glycopeptide mixture was separated with RP-HPLC and analysed online with electrospray mass spectrometry. The m/z spectra of the glycostructure containing peptide were integrated, the results are given in Table 2.

**Table 2**

| **Relative amount of glycosylation variants** | | | | | |
|---|---|---|---|---|---|
| **Clone No.** | **G0 [%]** | **G1 [%]** | **G2 [%]** | **NonFuc[%]** | **Man¹ [%]** |
| 1 | 38,4 | 51,4 | 10,2 | 0,1 | 0,5 |
| 2 | 44,3 | 47,6 | 8,1 | 0,1 | 0,6 |
| 3 | 42,8 | 48,7 | 8,5 | 0,2 | 0,8 |
| 4 | 49,2 | 43,6 | 7,2 | 0,3 | 1,2 |
| 5 | 62,7 | 33,0 | 4,3 | 0,6 | 1,0 |
| 6 | 60,4 | 35,5 | 4,2 | 0,5 | 1,2 |
| 7 | 40,4 | 49,8 | 9,8 | 0,3 | 0,6 |
| 8 | 46,9 | 45,9 | 7,3 | 0,3 | 1,1 |

| | | | | | |
|---|---|---|---|---|---|
| Man: High Mannose structures bearing four and five mannose residues respectively. G0, G1, G2: reduced heavy chains with fucosylated biantennary complex type carbohydrate with 1, 2 or 3 terminal galactose residues. nonFuc: reduced heavy chains with biantennary complex type carbohydrate without fucose. | | | | | |

The CHO cell line clone 5 (hu MAb<IGF-1R>B1-4E10_9-16) was deposited, under the Budapest Treaty on the international recognition of the deposit of microorganisms for the purposes of patent procedure, with Deutsche Sammlung von Mikroorganismen und Zellkulturen GmbH (DSMZ), Germany, on June 21, 2006 under Accession No. DSM ACC 2795.

The media used for cultivation of the different clones were obtained from Hyclone (HyQ SFM4 CHO-Utility, used for clone 4-6) or Sigma (C-8862 used for clone 1-3 and 7).

LCMS peptide map analysis was performed by integration of the specific ion chromatograms of all charge states for all glycopeptides.

Bisecting GlcNac, NGNA und and high mannose were determined in same manner.

Bisecting GlcNac and NGNA were not detectable. Thus the amount of NGNA is 0.5% or lower, and is also 0.1% or lower. The amount of bisecting GlcNac is also 0.5% or lower, and 0.1 % or lower.

An exemplary calculation of glycosylation is shown in Table 3 (Table 3a: clone 3, Table 3b: clone 5; peptide comprising asn298, named H27).

**Table 3a**

| | **Area z=2** | **Area z=3** | **Area z=4** | **Sum** | **rel. amount%** |
|---|---|---|---|---|---|
| **H27_G0** | 616 | 198 | 0 | 814 | **28,7** |
| **H27_G1** | 734 | 425 | 0 | 1158 | **40,9** |
| **H27_G2** | 103 | 135 | 0 | 238 | **8,4** |
| **H27_G3** | 0 | 0 | 0 | 0 | **0,0** |
| **H27_G4** | 0 | 0 | 0 | 0 | **0,0** |
| **H27_G1_1NGNA** | 0 | 0 | 0 | 0 | **0,0** |
| **H27_G2_1NGNA** | 0 | 0 | 0 | 0 | **0,0** |
| **H27_G2_2NGNA** | 0 | 0 | 0 | 0 | **0,0** |
| **H27_G3_1NGNA** | 0 | 0 | 0 | 0 | **0,0** |
| **H27_G3_2NGNA** | 0 | 0 | 0 | 0 | **0,0** |
| **G0 minus GlcNAc and minus Man** | 0 | 57 | 0 | 57 | **2,0** |
| **G0 minus GlcNAc** | 330 | 0 | 0 | 330 | **11,7** |
| **G1 minus GlcNAc** | 208 | 0 | 0 | 208 | **7,4** |
| **Man5** | 22 | 0 | 0 | 22 | **0,8** |
| **G0 minus Fuc** | 5 | 0 | 0 | 5 | **0,2** |
| **G1 minus Fuc** | 0 | 0 | 0 | 0 | **0,0** |
| **Man4** | 0 | 0 | 0 | 0 | **0,0** |
| **total** | | | | 2833,15 | **100,00** |
| **rel. amount of glycostructures with NGNA** | | | | | **0,0** |
| **rel. amount of glycostructures with Galactoses (G3 und G4)** | | | | | **0,0** |
| **rel. amount of high mannose** | | | | | **0,8** |
| **Rel. amount of G0 minus Fuc and G1 minus Fuc** | | | | | **0,2** |
| Sum G0 | | | | | 42,4 |
| Sum G1 | | | | | 48,2 |
| Sum G2 | | | | | 8,4 |
| Total Sum | | | | | 99,0 |
| Related to 100 % G0-1-2 | | | | | |
| **G0** | | | | | **42,8** |
| **G1** | | | | | **48,7** |
| **G2** | | | | | **8,5** |
| | | | | | |
| Sum without Man | | | | | 99,2 |
| Sum G0/1 minus Fuc | | | | | 0,2 |
| **Relative amount without Fuc** | | | | | **0,2** |

| | | | | | |
|---|---|---|---|---|---|
| Area: peak area H27_G0 - H27_G4: Glycopeptide H27 (containing Asn298) with fucosylated biantennary complex type carbohydrate with x-terminal galactose (e.g. G4 with 4 galactose units) Relative amount without Fuc: percentage of Fuc related to all G0, G1, G2 without mannose(4 and 5) glycostructure (high mannose). H27_G1_1NGNA - H27_G3_2NGNA: Glycopeptide H27 (containing Asn298) with fucosylated biantennary complex type carbohydrate with x-terminal galactose units (e.g. G2 with 2 units) bearing one to two N-glycolyl-neuraminic acids. Relative amount without Fuc: percentage of Fuc related to all G0, G1, G2 without mannose(4 and 5) glycostructure (high mannose). | | | | | |

**Table 3b**

| Exemplary calculation of glycosylation (clone5) | | | | | |
|---|---|---|---|---|---|
| | **Area z=2** | **Area z=3** | **Area z=4** | **Sum** | **rel. amount [%]** |
| **G0**¹⁾ | 1108 | 318 | 0 | 1426 | **43,8** |
| **G1**¹⁾ | 579 | 319 | 0 | 897 | **27,6** |
| **G2**¹⁾ | 67 | 71 | 0 | 139 | **4,3** |
| **G3**¹⁾ | 0 | 0 | 0 | 0 | **0,0** |
| **G4**¹⁾ | 0 | 0 | 0 | 0 | **0,0** |
| **G1_1NGNA**²⁾ | 0 | 0 | 0 | 0 | **0,0** |
| **G2_1NGNA**²⁾ | 0 | 0 | 0 | 0 | **0,0** |
| **G2_2NGNA**²⁾ | 0 | 0 | 0 | 0 | **0,0** |
| **G3_1NGNA**²⁾ | 0 | 0 | 0 | 0 | **0,0** |
| **G3_2NGNA**²⁾ | 0 | 0 | 0 | 0 | **0,0** |
| **G0-GlcNAc-Man**³⁾ | 0 | 95 | 0 | 95 | **2,9** |
| **G0-GlcNAc**³⁾ | 485 | 0 | 0 | 485 | **14,9** |
| **G1-GlcNAc**³⁾ | 159 | 0 | 0 | 159 | **4,9** |
| **Man5⁴⁾** | 32 | 0 | 0 | 32 | **1,0** |
| **G0-Fuc⁵⁾** | 11 | 0 | 0 | 11 | **0,3** |
| **G1-Fuc⁵⁾** | 9 | 0 | 0 | 9 | **0,3** |
| **Man4⁴⁾** | 0 | 0 | 0 | 0 | **0,0** |
| **Total** | | | | 3253,88 | **100,00** |
| | | | | | |
| **G0** | | | | | **62,7** |
| **G1** | | | | | **33,0** |
| **G2** | | | | | **4,3** |
| **glycostructures without fucose** | | | | | **0,6** |
| **glycostructures bearing NGNA** | | | | | **0,0** |
| **glycostructures bearing additional hexoses (G3+G4)** | | | | | **0,0** |
| **high mannose glycostructures** | | | | | **1,0** |

| | | | | | |
|---|---|---|---|---|---|
| ¹⁾ fucosylated biantennary compley type glycostructure with x-terminal galactose (0, 1, 2, 3 and 4 respectively) ²⁾ fucosylated biantennary compley type glycostructure with x-terminal galactose (0, 1, 2, 3 and 4 respectively) with additional n-glycolyl neuraminic acid residues ³⁾ fucosylated biantennary compley type glycostructures (mainly artefacts of the method) ⁴⁾ high Mannose structures bearing four or five mannose residues respectively ⁵⁾ non-fucosylated glycostructures | | | | | |

### Example 4

### Determination of antibody mediated effector functions by anti-IGF-IR HuMAbs

In order to determine the capacity of the generated HuMAb antibodies to elicit immune effector mechanisms, antibody-dependent cell cytotoxicity (ADCC) studies were performed.

To study the effects of the antibodies in ADCC, DU145 prostate cancer cells (HTB-81 ATCC; 1 × 106 in 2 to 4 ml RPMI-FM) expressing IGF-IR were labeled with 1 µl bis(acetoxymethyl) 2,2':6',2"-terpyridine-6,6"-dicarboxylate (BATDA) solution for 25 minutes at 37°C in a cell incubator. Cells were washed four times with 10 ml of RPMI-FM and spun for 10 minutes at 200 xg with brake. Afterwards, cells were adjusted to a concentrations of 1 × 10⁵ cells per ml. 5,000 cells were plated per well in a round bottom plate corresponding to a volume of 50 µl. HuMAb antibodies were added at a final concentration ranging from 25-0.1 ng/ml in a volume of 50 µl cell culture medium. Subsequently, 50 µl of effector cells, PBMC freshly isolated from whole blood or purified effector cells from buffycoats, were added at an E:T ratio in the range of 25:1. The plates were centrifuged immediately for 1 minute at 200 xg with brake, and incubated for 2 hours at 37°C. After incubation the cells were spun down for 10 minutes at 200 xg and 20 µl of supernatant were transferred to an Optiplate 96-F microtiterplate. 200 µl of Europium solution (at room temperature) were added and the mixture was incubated for 15 minutes on a shaker. Resulting fluorescence was measured in a time-resolved fluorometer using the EU-TDA protocol from Perkin Elmer.

The magnitude of cell lysis by ADCC is expressed as % of the maximum release of TDA from the target cells lysed by detergent corrected for spontaneous release of 2,2':6',2"-terpyridine-6,6"-dicarboxylate (TDA) from the respective target cells. As reference standard of an antibody showing "no ADCC" is used a (monoclonal) antibody against KLH (keyhole limpet hemocyanin) of the same IgG type or an IgG mixture isolated from about 35.000 donors ("Redimune"). A 75% fucose free antibody against IGF-IR was used as positive control. An antibody according to the invention showed a TDA release which is within 3xSD of the TDA release of the standard antibody (Fig. 1).

### Example 5

### Determination of the affinity of anti-IGF-IR antibodies to IGF-IR

| | |
|---|---|
| Instrument: | BIACORE^{®} 3000 |
| Chip: | CM5 |
| Coupling: | amine coupling |
| Buffer: | HBS (HEPES, NaCl), pH 7.4, 25°C |

For affinity measurements anti human FCy antibodies (from rabbit) have been coupled to the chip surface for presentation of the antibody against IGF-IR. IGF-IR extracellular domain was added in various concentrations in solution. Association was measured by an IGF-IR-injection of 3 minutes; dissociation was measured by washing the chip surface with buffer for 5 minutes. The affinity data for antibodies 18 and 22 are shown in Table 4.

**Table 4**

| Affinity data measured by SPR (BIACORE^{®} 3000) | | | |
|---|---|---|---|
| **Antibody** | **ka (1/Ms)** | **kd (1/s)** | **KD (M)** |
| 18 | 1.49 × 10⁵ | 1.03 × 10⁻⁷ | 6.95 × 10⁻¹³ |
| 22 | 1.47 × 105 | 9.64 × 10⁻⁵ | 6.56 × 10⁻¹⁰ |

### Example 6

### Inhibition of IGF-I and IGF-II binding to tumor cells expressing IGF-IR

In order to determine the ability of the antibody of the invention to block binding of the ligands IGF-I and IGF-II to the IGF-I receptor (IGF-IR), competition experiments with radioactively labeled ligand peptides were performed.

Human tumor cells (HT29, NCI H322M, 0.5 to 1 × 10⁵/ml) were plated in RPMI 1640 medium (PAA, Cat. No. E15-039) supplemented with 2 mM L-Glutamin, 1x non-essential amino acids (Gibco, Cat. No. 11140-035), 1 mM sodium pyruvate (Gibco, Cat. No. 11360-039) and 10% heat inactivated FCS (PAA, Cat. No. A15-771). Six bottles in the T175 format were inoculated with 20 ml cells in the respective medium for each experiment and cultivated for two days at 37°C and 5% CO₂ to obtain confluent cell monolayers.

To collect individual cells, 2 ml of 1x Trypsin/EDTA (Gibco, Cat. No. 25300-054) per T175 flask were added and detachment of cells monitored with a Zeiss Axiovert25 microscope. The cells were collected and medium with 10% FCS as described before was added to a total volume of 50 ml. Cells were reisolated by centrifugation for 10 minutes at 1000 rpm (Heraeus sepatech, Omnifuge 2.0 RS) and resuspended in 50 ml of binding buffer (120 mM NaCl, 5 mM KCI, 1.2 mM MgSO₄, 1 mM EDTA, 10 mM D(+)glucose, 15 mM NaAc, 100 mM Hepes pH 7.6, 1% BSA). Cells were counted, reisolated by centrifugation and adjusted with binding buffer to 1 × 10⁶ cells/ml.

I¹²⁵-labeled IGF-I and IGF-II peptides (Amersham, ∼2000 Ci/mmol, Cat. No. IM172 and IM238), solubilized in 0.1% CH₃COOH, were diluted in binding buffer to a final activity of 4 × 10⁵ counts/(minute × ml). 75 µl of antibody at the specified concentrations together with 25 µl of prediluted I¹²⁵-labeled IGF-I or IGF-II peptide was added to 200 µl of cell suspension and incubated for 3,5 h at 4°C. Cells were reisolated by centrifugation for 5 minutes at 2000 rpm (Eppendorf, 5415C) and supernatant removed. After washing two times in 1 ml binding buffer, cells were resuspended in 1 ml binding buffer and transferred to scintillation tubes. The amount of radioactive peptide bound to the cell surface receptors was measured on a scintillation counter.

The average IC₅₀ value for antibody 18 is 0.3 nM. No detectable inhibition for IGF-II binding could be observed.

### Example 7

### Antibody competition assay for IGF-IR binding

For an epitope mapping of anti-IGF-IR monoclonal antibodies a similar format as for affinity measurement (Example 5) was selected, but IGF-IR was pre-incubated for at least 0.5 hours at RT with the antibody in solution. This mixture was injected and IGF-IR binding (or inhibition) was detected. This assay allows measuring the reciprocal inhibitory activity of monoclonal antibodies for IGF-IR binding. It was found that the antibodies of the invention compete for binding to IGF-IR with αIR3, an antibody which is known to bind to aa 217-274 (Gustafson, T.A., and Rutter, W.J., J. Biol. Chem. 265 (1990) 18663-18667).

### Example 8

### Inhibition of IGF-I mediated phosphorylation of IGF-IR and Akt/PKB

In order to determine the ability of the antibody of the invention to inhibit activation and phosphorylation of the IGF-I receptor (IGF-IR), competition experiments were performed with IGF-I peptide and subsequent Western blotting analysis with antibodies specific for phosphorylated tyrosine.

Human tumor cells (HT29, NCI H322M, 5 × 10⁴/ml) were plated in RPMI 1640 medium (PAA, Cat. No. E15-039) supplemented with 2 mM L-Glutamin, 1x non-essential aminoacids (Gibco, Cat. No. 11140-035), 1 mM sodium pyruvate (Gibco, Cat. No. 11360-039) and 0.5% heat inactivated FCS (PAA, Cat. No. A15-771). For determination of IC₅₀ values, 12 well plates were inoculated with 1ml cells in the respective medium for each experiment and cultivated for two days at 37°C and 5% CO₂.

After 48 hours of cultivation with low serum medium, the medium was carefully removed and replaced by different concentrations of antibody diluted in the respective medium. After 5 minutes incubation at 37°C and 5% CO₂ IGF-I peptide was added at a final concentration of 2 nM and cells were again incubated for 10 minutes under the conditions mentioned above. The medium was carefully removed by aspiration and 100 µl of cold lysis buffer was added per well (50mM Hepes pH 7.2, 150 mM NaCl, 1mM EGTA, 10% glycerol, 1% Triton^{®}-X100, 100mM NaF, 10 mM Na₄P₂O₇, Complete^{®} protease inhibitor). The cells were detached using a cell scraper (Corning, Cat. No. 3010) and well contents transferred to Eppendorf reaction tubes. Cell fragments were removed by centrifugation for 10 minutes at 13000 rpm and 4°C and half of the supernatant was added to 2x Laemmli sample buffer in a 1:1 (v/v) ratio. For immunoprecipitation of IGF-IR, the remaining supernatant of cell lysates underwent a clearifying spin (10 minutes at 13000 rpm and 4°C) right before 1µl of an polyclonal antibody against IGF-IRß (C-20, Santa Cruz Biotechnologies) or a murine monoclonal antibody (IgG1) which recognizes an epitope within amino acids 440-586 of the extracellular domain (α-chain) of the human IGF Type 1 Receptor was added (mAb 24-55, GroPep). After 2 hours incubation at 4°C in a rotating Eppendorf reaction tube, 25 µl Protein G Sepharose^{®} beads (Amersham Biosciences, Cat. No. 17-0618-01) were added followed by another incubation step of 1 hour at 4°C. The beads with bound antibody-protein-complexes were isolated by centrifugation (1 minute at 2000 rpm and 4°C) and washed three times with wash buffer (lysis buffer with only 0.1% Triton^{®}-X100). After boiling the beads in Laemmli sample buffer, cellular proteins were separated by SDS-PAGE and transferred to a nitrocellulose membrane (PROTRAN^{®} BA 85, Schleicher&Schuell) by semi-dry Western blotting.

A phosphotyrosine specific antibody (Upstate, clone 4G10, Cat. No. 05-321) was used to determine phosphorylation status of immunopurified IGF-IR. For the detection of phosphorylated Akt/PKB an antibody with specificity for phosphorylated Ser473 (Cell Signalling, Cat. No. 9271) was applied.

It was found that antibody 18 can inhibit IGF-1 mediated phosphorylation of IGF-1R and PKB with an IC₅₀ of 0.6 nM.

### Example 9

### Induction of antibody mediated downregulation of IGF-IR in-vitro

In order to detect effects of the antibody of the invention on the amount of IGF-I receptor (IGF-IR) in tumor cells, time-course experiments and subsequent western-blotting analysis with IGF-IR specific antibodies were performed.

Human tumor cells (HT29, 5 × 10⁴ cells/ml) in RPMI 1640 medium (PAA, Cat. No. E15-039) supplemented with 2 mM L-Glutamin, 1x non-essential aminoacids (Gibco, Cat. No. 11140-035), 1 mM sodium pyruvate (Gibco, Cat. No. 11360-039) and 10% heat inactivated FCS (PAA, Cat. No. A15-771). For each incubation period one 12 well plate was inoculated with 1 ml cells in the respective medium for each experiment and cultivated for 24 hours at 37°C and 5% CO₂.

The medium was carefully removed and replaced by different concentrations of antibody diluted in the respective medium. In two control wells, medium was replaced by either medium without antibody or medium with a control antibody (AB-1, Oncogene, Cat. No. GR11). Cells were incubated at 37°C and 5% CO₂ and individual plates were taken out for further processing after 15 minutes, 24 hours and 48 hours.

The medium was carefully removed by aspiration and 100 µl of cold lysis buffer was added per well (50mM Hepes pH 7.2, 150 mM NaCl, 1mM EGTA, 10% glycerol, 1% Triton^{®}-X100, 100mM NaF, 10 mM Na₄P₂O₇, Complete^{®} protease inhibitor). The cells were detached using a cell scraper (Corning, Cat. No. 3010) and well contents transferred to Eppendorf reaction tubes. Cell fragments were removed by centrifugation for 10 minutes at 13000 rpm and 4°C and the supernatant was added to 2x Laemmli sample buffer in a 1:1 (v/v) ratio. Cellular proteins were separated by SDS-PAGE and transferred to a nitrocellulose membrane (PROTRAN^{®} BA 85, Schleicher&Schuell, Cat. No. 10 401196) by semi-dry western-blotting.

An antibody specific for IGF-IR (C-20, Santa Cruz Biotechnologies, Cat. No. sc-713) was used to determine protein levels of IGF-IR.

Downregulation of IGF-IR induced by the antibody of the invention after less than 24 hours after addition of the antibody was observed.

### Example 10

### Inhibition of insulin binding to 3T3-cells expressing human insulin receptor

In order to determine whether the antibody of the invention also blocks binding of insulin to the insulin receptor (IR), competition experiments were performed with a radioactively labeled ligand peptide.

3T3 cells (1 × 10⁵/ml) expressing recombinantly high numbers (>10⁵) human IR were plated in MEM Dulbecco medium (DMEM) with high glucose (PAA, Cat. No. E15-009) supplemented with 2mM L-Glutamin (Gibco, Cat. No. 25030-024) and 10% heat inactivated FCS (PAA, Cat. No. A15-771). Six bottles in the T175 format were inoculated with 20 ml cells in the respective medium for each experiment and cultivated for two days at 37°C and 5% CO₂ to obtain confluent cell monolayers.

To collect individual cells, 2 ml of 1x Trypsin/EDTA (Gibco, Cat. No. 25300-054) per T175 flask were added and detachment of cells monitored with a microscope. The cells were collected and medium with 10% FCS as described before was added to a total volume of 50 ml. Cells were reisolated by centrifugation for 10 minutes at 1000 rpm and resuspended in 50 ml of binding buffer (120 mM NaCl, 5 mM KCI, 1.2 mM MgSO₄, 1 mM EDTA, 10 mM D(+)glucose, 15 mM NaAc, 100 mM Hepes pH 7.6, 1% BSA). Cells were counted, reisolated by centrifugation and adjusted with binding buffer to 1 × 10⁶ cells/ml.

I¹²⁵-labeled insulin peptide (Amersham, Cat. No. IM166, ∼2000 Ci/mmol), solubilized in 0.1% CH₃COOH, were diluted in binding buffer to a final activity of 4*10⁵ counts/(minute*ml). 75 µl of antibody together with 25 µl of prediluted I¹²⁵-labeled insulin peptide was added to 200 µl of cell suspension (final antibody concentration 200 nM) and incubated for 3,5 h at 4°C. Cells were reisolated by centrifugation for 5 minutes at 2000 rpm and supernatant was removed. After washing two times in 1 ml binding buffer, cells were resuspended in 1 ml binding buffer and transferred to scintillation tubes. The amount of radioactive peptide bound to the cell surface receptors was measured on a scintillation counter.

The results demonstrate that the antibody of the invention does not interfere with binding of insulin ligand to the insulin receptor.

### Example 11

### No stimulation of IGF-IR and Akt/PKB phosphorylation

In order to exclude IGF-IR stimulating activities of the antibody of the invention, phosphorylation of IGF-IR was determined in the absence of IGF-I ligand but in the presence of the antibody of the invention and a reference antibody (αIR3, Oncogene, Germany). This was performed by a western-blotting analysis with phosphorylation-state specific antibodies. 3T3 cells (ATCC CRL 1658) transfected with IGF-IR (5*10⁴cells/ml, Pietrzkowski, Z., et al., Cell Growth Differ. 4 (1992) 199-205) were plated in MEM. Dulbecco medium (DMEM) with high glucose (PAA, CatNo. E15-009) supplemented with 2mM L-Glutamin (Gibco, CatNo. 25030-024) and 0.5% heat inactivated FCS (PAA, CatNo. A15-771) or human tumor cells (HT29, NCI H322M, 5*10⁴/ml) in RPMI 1640 medium (PAA, CatNo. E15-039) supplemented with 2 mM L-Glutamin, 1x non-essential aminoacids (Gibco, CatNo. 11140-035), 1 mM sodium pyruvate (Gibco, CatNo. 11360-039) and 0.5% heat inactivated FCS (PAA, CatNo. A15-771). For determination of IC₅₀ values, 12 well plates were inoculated with 1 ml cells in the respective medium for each experiment and cultivated for two days at 37°C and 5% CO₂.

After 48 hours of cultivation with low serum medium, the medium was carefully removed and replaced by different concentrations of antibody diluted in the respective medium. Cells were incubated for 15 minutes under the conditions mentioned above. The medium was carefully removed by aspiration and 100 µl of cold lysis buffer was added per well (50mM Hepes pH 7.2, 150 mM NaCl, 1mM EGTA, 10% glycerol, 1% Triton-X100, 100mM NaF, 10 mM Na₄P₂O₇, Complete™ protease inhibitor). The cells were detached using a cell scraper (Corning, CatNo. 3010) and well contents transferred to Eppendorf reaction tubes. Cell fragments were removed by centrifugation for 10 minutes at 13000 rpm and 4°C (Eppendorf centrifuge 5415R) and half of the supernatant was added to 2x Laemmli sample buffer in a 1:1 (v/v) ratio. For immunoprecipitation of IGF-IR, the remaining supernatant of cell lysates underwent a clearifying spin (10 minutes at 13000 rpm and 4°C) right before 1 µl of an antibody against IGF-IR was added (C-20, Santa Cruz Biotechnologies, CatNo. sc-713 or mAb 24-55, GroPep, CatNo. MAD1). After 2 hours incubation at 4°C in a rotating Eppendorf reaction tube, 25 µl Protein G Sepharose™ beads (Amersham Biosciences, CatNo. 17-0618-01) were added followed by another incubation step of 1 hour at 4°C. The beads with bound antibody-protein-complexes were isolated by centrifugation (1 minute at 2000 rpm and 4°C) and washed three times with wash buffer (lysis buffer with only 0.1% Triton-X100). After boiling the beads in Laemmli sample buffer, cellular proteins were separated by SDS-PAGE and transferred to a nitrocellulose membrane (PROTRAN BA 85, Schleicher&Schuell, CatNo. 10 401196) by semi-dry western-blotting.

A phosphotyrosine specific antibody (Upstate, clone 4G10, CatNo. 05-321, recognizing tyrosine-phosphorylated proteins) was used to determine phosphorylation status of immunopurified IGF-IR. For the detection of phosphorylated Akt/PKB an antibody against Akt1 with specificity for phosphorylated Ser473 (Cell Signalling, CatNo. 9271) was applied.

It was observed that the Akt/PKB kinase downstream in the signalling pathway of IGF-IR was significantly activated by the reference antibody at concentrations higher than 5 nM but not by the antibody of the invention at concentrations up to 10.000 nM.

### Example 12

### Induction of receptor down-regulation in H322M xenograft models

Tumors were induced in nude mice and treated once with different concentrations of the antibody of the invention. 24 hours after treatment the tumors were extracted and homogenized under liquid nitrogen. Cold lysis buffer was added (50mM Hepes pH 7.2, 150 mM NaCl, 1mM EGTA, 10% glycerol, 1% Triton-X100, 100mM NaF, 1 mM Na₃VO₄, 10 mM Na₄P₂O₇, Complete™ protease inhibitor, 1mM PMSF) in a buffer-volume to tumor-weight ratio of 3:1 and thoroughly mixed with the thawing tumor homogenate. After solubilizing the tissue for 15 minutes on ice, insoluble fragments were removed by centrifugation for 10 minutes at 13000 rpm and 4°C (Eppendorf centrifuge 5415R). The protein concentration of the samples was determined with the Micro BCA™ Reagents (Pierce) and lysis buffer was added to adjust equal concentrations. Part of the supernatant was added to 2x Laemmli sample buffer in a 1:1 (v/v) ratio. Cellular proteins were separated by SDS-PAGE and transferred to a nitrocellulose membrane (PROTRAN BA 85, Schleicher&Schuell, CatNo. 10 401196) by semi-dry western-blotting. An IGF-IR specific antibody (C-20, Santa Cruz Biotechnologies, CatNo. sc-713) was used to detect IGF-IR.

Upon treatment with the antibody of the invention, a concentration dependent decrease of IGF-IR levels with an estimated EC50 at 0.6 mg/kg was observed.

### Example 13

### Growth inhibition of H322M tumors

The effects of antibody 18 in vivo was investigated by inducing tumors in athymic nude mice according to established methods. Human H322M NSCLC cells were coinjected together with Matrigel subcutaneously into 6-7 week-old athymic nu mice (nu/nu). For that purpose, 5 × 10⁶ H322M cells were concentrated in 100µl culture medium and mixed with 100 µl Matrigel. 200µl of this mixture were injected into the right flanks of the mice. Tumor volume was calculated by measuring tumor diameters with Vernier calipers twice a week according to the formula first published by Geran et al. ("Protocols for screening chemical agents and natural products against animal tumors and other biological systems", Cancer Chemother. Rep. 11.301, 1972) where tumor volume [mg] = (length × (width)²).

Antibody was administered intraperitoneally (i.p.) at 10ml/ kg. Treatment was started with doubled doses of the antibody administered in doubled volumes. Tumors were induced in nude mice as described above. After tumors had grown to an average volume of 160 mg, mice were treated intraperitoneally six times once a week with 6, 0.6 and 0.06 mg/ kg of antibody as consecutive doses starting with 12, 1.2 and 0.12 mg/ kg as loading dose given once on the first day of treatment. The experiment demonstrates that blocking of the IGF-IR axis by rhu anti-IGF-IR mAb 18 results in antitumoral efficacy when administered as a single agent at 6 and 0.6 mg/kg. In contrast, 0.06 mg/kg had no effect on tumor growth.

In addition antibody 18 was tested in combination with gemcitabine in the same model. Tumors were induced as described above and treatment was initiated when tumors had established and grown to 170mm³ average in all groups. Antibody was administered once a week i.p. at 6 and 0.6 mg/kg and in combination with 62 mg/ kg of gemcitabine at 0.6 mg. Gemcitabine was administered one cycle i.e. every third day for four times in total. Treatment was started by administering doubled doses of the antibody. The experiment demonstrated that treatment with antibody 18 administered once every seven days inhibits tumor growth by itself and enhances the effectiveness of gemcitabine, a known antimetabolic compound.

### Example 14

### Growth inhibition of 3T3 tumors

Tumors were induced in nude mice essentially as described in Example 15 except that murine 3T3 fibroblasts overexpressing the human IGF-IR were used. Mice with established tumors of approximately 180 mg were treated intraperitoneally once weekly for seven times with 18, 6 or 0.6 mg/kg of antibody 18. Treatment was started with doubled doses of antibody given as loading dose (36, 12 and 1.2 mg/kg). The experiment demonstrates that by treatment with the antibody, tumor growth can be delayed when administered at 18 and 6 mg/kg once weekly.

### Example 15

### Induction of antibody mediated downregulation of IGF-1R in vitro

In order to detect effects of the antibody of the invention on the amount of IGF-I receptor (IGF-IR) in tumor cells, time-course experiments and subsequent western-blotting analysis with IGF-IR specific antibodies were performed.

Human tumor cells (HT29, 5 × 10⁴ cells/ml) in RPMI 1640 medium (PAA, Cat. No. E15-039) supplemented with 2 mM L-Glutamin, 1x non-essential aminoacids (Gibco, Cat. No. 11140-035), 1 mM sodium pyruvate (Gibco, Cat. No. 11360-039) and 10% heat inactivated FCS (PAA, Cat. No. A15-771). For each incubation period one 12 well plate was inoculated with 1ml cells in the respective medium for each experiment and cultivated for 24 hours at 37°C and 5% CO₂.

The medium was carefully removed and replaced by different concentrations of antibody diluted in the respective medium. In two control wells, medium was replaced by either medium without antibody or medium with a control antibody (AB-1, Oncogene, Cat. No. GR11). Cells were incubated at 37°C and 5% CO₂ and individual plates were taken out for further processing after 15 minutes, 24 hours and 48 hours.

The medium was carefully removed by aspiration and 100 µl of cold lysis buffer was added per well (50mM Hepes pH 7.2, 150 mM NaCl, 1mM EGTA, 10% glycerol, 1% Triton®-X100, 100mM NaF, 10 mM Na₄P₂O₇, Complete^{®} protease inhibitor). The cells were detached using a cell scraper (Corning, Cat. No. 3010) and well contents transferred to Eppendorf reaction tubes. Cell fragments were removed by centrifugation for 10 minutes at 13000 rpm and 4°C and the supernatant was added to 2x Laemmli sample buffer in a 1:1 (v/v) ratio. Cellular proteins were separated by SDS-PAGE and transferred to a nitrocellulose membrane (PROTRAN^{®} BA 85, Schleicher&Schuell, Cat. No. 10 401196) by semi-dry western-blotting.

An antibody specific for IGF-IR (C-20, Santa Cruz Biotechnologies, Cat. No. sc-713) was used to determine protein levels of IGF-IR.

Downregulation of 50% or more IGF-IR induced by the antibody of the invention after 24 hours after addition of the antibody was observed.

## Claims

1. Antibody binding to IGF-IR, being of human IgG1 or IgG3 type and being glycosylated with a sugar chain at Asn297, said antibody being **characterized in that** the amount of fucose within said sugar chain is at least 99% ("completely fucosylated") , and in addition the amount of NGNA is 1% or less and/ or the amount of N-terminal alpha-1,3-galactose is 1% or less.

2. Antibody according to claim 1, **characterized in that** the amount of NGNA is 0.5% or less.

3. Antibody according to claims 1 or 2, **characterized in that** the amount of N-terminal alpha 1,3 galactose is 0.5% or less.

4. Antibody according to claims 1 to 3, **characterized in that** the antibody is a chimeric, humanized or human antibody.

5. Antibody according to claims 1 to 4, **characterized in that** said antibody shows one or more properties selected from the group consisting of:
a) shows a ratio of IC₅₀ values of inhibition of the binding of IGF-I to IGF-IR to the inhibition of binding of IGF-II to IGF-IR of 1:3 to 3:1,
b) inhibits for at least 80%, preferably at least 90%, at a concentration of 5 nM IGF-IR phosphorylation in a cellular phosphorylation assay using HT29 cells in a medium containing 0.5% heat inactivated fetal calf serum (FCS) when compared to such an assay without said antibody.
c) shows no IGF-IR stimulating activity (no signaling , no IGF-1 mimetic activity) measured as PKB phosphorylation at a concentration of 10 µM in a cellular phosphorylation assay using 3T3 cells providing 400,000 to 600,000 molecules IGF-IR per cell in a medium containing 0.5% heat inactivated fetal calf serum (FCS) when compared to such an assay without said antibody.

6. Antibody according to claims 1 to 5, **characterized by** an affinity of about 10⁻¹³ to 10⁻⁹ M (K_{D}).

7. Antibody according to claims 1 to 5, **characterized by** comprising as complementarity determining regions (CDRs) having the following sequences:
a) an antibody heavy chain comprising as CDRs CDR1 (aa 31-35), CDR2 (aa 50-66) and CDR3 (aa 99-107) of SEQ ID NO:1 or 3;
b) an antibody light chain comprising as CDRs CDR1 (aa 24-34), CDR2 (aa 50-56) and CDR3 (aa 89-98) of SEQ ID NO:2 or 4.

8. The use of an antibody according to claims 1 to 7 for the manufacture of a pharmaceutical composition.

9. A pharmaceutical composition containing an antibody according to claims 1 to 7 in a pharmaceutically effective amount.

10. Method for the manufacture of a pharmaceutical composition comprising a pharmaceutically effective amount of an antibody according to claims 1 to 7.

11. Method for the treatment of a patient in need of an antitumor therapy, **characterized by** administering to the patient a pharmaceutically effective amount of an antibody according to claims 1 to 7.

12. Method according to claim 15, **characterized in that** the antibody is administered in combination with a cytotoxic agent, a prodrug thereof or a cytotoxic radiotherapy.

13. CHO cell capable of recombinantly expressing an antibody according to claims 1 to 7.
